(19) Europäisches Patentamt
European Patent Office
Office européen des brevets

(11) Publication number: **0 471 081 A1**

(12) **EUROPEAN PATENT APPLICATION published in accordance with Art. 158(3) EPC**

(21) Application number: **91903824.0**

(22) Date of filing: **18.02.91**

(86) International application number:
**PCT/JP91/00199**

(87) International publication number:
**WO 91/13165 (05.09.91 91/21)**

(51) Int. Cl.5: **C12P 19/44**, C09K 15/08, //C12R1/91

(30) Priority: **20.02.90 JP 37484/90**

(43) Date of publication of application:
**19.02.92 Bulletin 92/08**

(84) Designated Contracting States:
**CH DE FR GB IT LI**

(71) Applicant: **KABUSHIKI KAISHA KOBE SEIKO SHO**
**3-18 Wakinohama-cho 1-chome Chuo-ku**
**Kobe 651(JP)**

(72) Inventor: **MIMURA, Akio**
**435-7, Miyashita, Fuji-shi**
**Sizuoka-ken 416(JP)**
Inventor: **TAKEBAYASHI, Keiichi**
**Koberukohaitsu 303, 18-5, Kasuga 2-chome,**
**Tsukuba-shi Ibaragi-ken 305(JP)**
Inventor: **TAKAHARA, Yoshimasa**
**29-8, Yatsu 5-chome**
**Narashino-shi, Chiba-ken 275(JP)**
Inventor: **OSAWA, Toshihiko**
**7-9-8, Oshizawadai, Kasugai-shi**
**Aichi-ken 487(JP)**

(74) Representative: **Lewin, John Harvey**
**Elkington and Fife Prospect House 8**
**Pembroke Road**
**Sevenoaks, Kent TN13 1XR(GB)**

(54) **PRODUCTION AND USE OF ANTIOXIDANT GLYCOSIDE.**

(57) A process for producing a glycoside of formula (I) from a culture of proliferative cells derived from a grown plant of sesame, *sesamum indicum L.* The use of sesame makes it possible to produce the glycoside in a large amount. An antioxidant containing the glycoside of formula (I) as the active ingredient, which is harmless and particularly effective in inhibiting the oxidation of food, drug, cosmetic, etc.



Rank Xerox (UK) Business Services

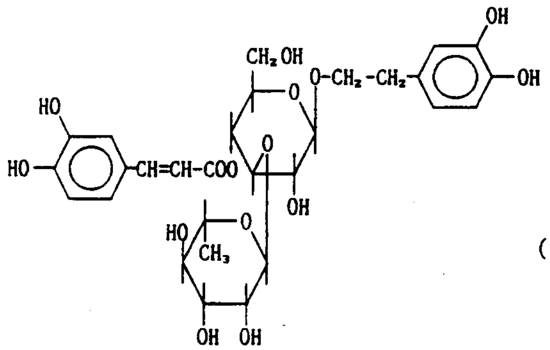

(I)

Field of the Invention

The present invention relates to a process for preparing a glycoside extracted from callus artificially derived from the plant body of sesame (Sesamum indicum L.) which has an antioxidative activity and also relates to use thereof. Background

In general, foodstuffs are prepared from agricultural products, marine products, livestock products, etc. However, during the course of storage or preservation processing of raw food materials or products, raw food materials or products are deteriorated by contamination and putrefaction with microorganisms or by chemical or physical action to reduce their commercial value. Therefore, a variety of food additives have been developed and at the same time, methods for a temperature treatment, an oxygen absorption treatment, vacuum packaging, low temperature storage, a radiation treatment, etc. have been developed and practically used.

The most serious problem in such deterioration of food materials or products is oxidation or peroxidation of food ingredients with oxygen in the air. Oxygen maintains life of the living thing through respiration. On the other hand, it is known that oxygen is a very reactive compound so that it reacts with various ingredients in food to oxidize or peroxidize the ingredients thereby to not only reduce their commercial value but also form injurious materials in food. It is reported that for example, nutritionally required unsaturated fatty acids such as linoleic acid, linolenic acid, etc., which are contained in foods, are readily peroxidized by oxygen in the air to form peroxidized fatty acids or reactive radicals (free radicals) and at the same time, form carcinogenic substances such as malone dialdehyde, etc. It is also reported that peroxidized lipids formed by peroxidation of unsaturated fatty acid molecules in lipids modify protein or nucleic acid in vivo to cause the living body in its carcinogenic action, etc. ("Mutagen and Toxicity", vol. 5, page 243 (1982), "Packaging of Food", vol. 17, page 106 (1986)).

In order to prevent such peroxidation of lipids, techniques for packaging such as removal of oxygen in the package with a free oxygen absorber, vacuum packaging, nitrogen gas substitution packaging, etc. have been used. On the other hand, synthetic antioxidants, for example, butylhydroxyanisole (BHA), butylhydroxytoluene (BHT), etc. have been generally used, backed by development of chemical industries. However, as use of such synthetic antioxidants increases, food pollution increases and a serious problem occurs in view of safety. It is thus the actual situation that the consumer's rejection against synthetic antioxidants has been increasing and its amount of use decreases.

On the other hand, it is considered that peroxides or carcinogenes formed in animal living body would adversely affect animal cells due to the toxic action of oxygen as described above. Such peroxidation of vital components with oxygen would be correlated to aging of cells and thus to a life span (theory of free radical aging). Therefore, highly safe antioxidants derived from the natural world have been greatly expected to be substances for supporting the antioxidative protective mechanism in vivo, not only in foodstuffs, especially health maintaining foods or nutrient foods, but also in the technical fields of medicines and cosmetics.

However, the only natural antioxidants that have been expected to be practically used in place of synthetic antioxidant involving problems in food pollution are vitamin C prepared by chemical synthesis and vitamin E (tocopherol) extracted and purified from natural products.

In order to suitably use such antioxidants derived from the natural world for use in foods, drugs, cosmetics, etc., it is important to find natural antioxidants having different properties and utilize the antioxidants under such conditions that their characteristics are exhibited.

A variety of compounds having an antioxidative activity are contained in spices derived from plants and, spices have been added to food as having an action of preserving foods (Packaging of Food, vol. 19, No. 1, page 97, 1987). However, most of spices exhibit a strong flavor or color. In order to add these spices to foods, drugs, cosmetics, etc. as antioxidants or the like, such properties have automatically limited their use range.

Vitamin C is insoluble in fat or lipids in vivo since it is a water soluble substance. On the other hand, vitamin E is insoluble in an aqueous solution such as blood, etc. and accumulated in lipids in vivo, since it is fat-soluble. Such properties of extreme water solubility and fat solubility are not considered to be necessarily advantageous, in the case of foods, drugs, cosmetics, etc. applied to the living body. In order to exhibit their antioxidative activity appropriately in any of lipids and aqueous solutions in vivo, natural substances having an intermediate property between water solubility and fat solubility are advantageous.

In addition to vitamin C or vitamin E, extensive investigations have been made on properties of natural antioxidants derived from spice plants, and reports are made thereon.

However, natural antioxidants other than natural vitamin E, vitamin C, etc., that are expected to be used in place of synthetic antioxidants involving problems in food pollution have not been practically utilized,

because their origins are plants or animals affected by natural conditions such as weather, etc. and it is thus difficult to stably supply them, their contents are extremely a trace amount, extraction may be made only with extreme difficulty, and components change during extraction.

A potent antioxidative activity is noted in sesame oil obtained from seeds of Sesamum indicum L. by lignan compounds modified during the course of purification of sesame oil. Thus, sesame oil has been given an important position as an excellent edible oil which is not deteriorated by oxidation ("Grand Modern Encyclopedia", Gakushu Kenkyusha (January 1, 1979), pages 123-124).

On the other hand, it is already known that not only sesame oil is contained in Sesamum indicum L. but also antioxidants such as vitamin E or lignan compounds, etc. are contained in the plant body of sesame (Agricultural and Biological Chemistry, vol. 49, page 301 (1985)), Journal of the Japanese Food Industry Association, vol. 32, page 407 (1985)). However, it is quite unknown or not even suggested that growing cells from sesame plants, especially cells grown at high temperature can be industrially cultivated, a glycoside substance having a potent antioxidative activity is contained in these growing cells, and the substance can be efficiently extracted and produced industrially without causing any denaturation or deterioration.

Therefore, the present inventors invented a process for systematically extracting and producing useful components in large quantities industrially, not extracting useful components from sesame plants themselves, and filed a patent application directed to the invention. A gist of the invention described above lies in preparing cells grown at high temperature by subjecting a callus of the plant body, not the plant body, to conditioned culture at high temperature and further lies in preparing the antioxidant-containing cells by culturing the cells grown at high temperature. That is, by establishing quite a novel process for induction culture of sesame plant callus, especially cultivation at high temperature, the previous invention enabled to extract the useful components contained in Sesamum indicum L. in large quantities. This patent application was laid open to the public on August 29, 1990 as Japanese Published Unexamined Patent Application No. 1-215282.

The present inventor has made further investigations and succeeded in culturing the cells grown at high temperature induced from the plant body of sesame in a medium and extracting a glycoside shown by the following structural formula (I) which has an antioxidative activity. However, the substance of the structural formula (I) was known by Phytochemistry, vol. 25, No. 7, pages 1633-1636, 1986.

(Disclosure of the Invention)

That is, the present invention relates to a process for extracting a glycoside of the following structural formula (I) having an antioxidative activity from the culture broth of callus obtained by inducing the plant body of sesame and also relates to an antioxidant comprising the glycoside as an effective component.

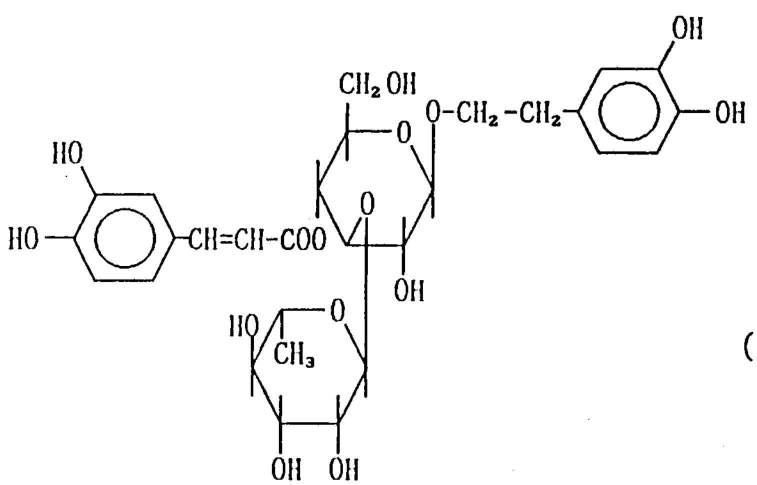

(I)

BRIEF DESCRIPTION OF THE DRAWINGS

Fig. 1 is a graph showing relationship between growth of callus and culture temperature.

Fig. 2 is a chromatogram of a crude sample by liquid chromatography showing a composition of components contained in the crude extract eluted by adsorption chromatography. Substance 1 corresponds to the peak obtained by elution for 6 minutes.

Fig. 3 shows a chromatogram of Substance 1 purified by liquid chromatography.

Fig. 4 is a UV absorption spectrum of Substance 1.

Fig. 5 is an analytical pattern of Substance 1 by high speed electron bombardment mass spectrum.

Fig. 6 is a spectrum of Substance 1 by nuclear magnetic resonance.

Fig. 7 is a spectrum of Substance 1 by protonic nuclear magnetic resonance.

Fig. 8 is a spectrum of Substance 1 by carbon 13 nuclear magnetic resonance.

Fig. 9 shows an antioxidative activity of crude extract from sesame culture cell by the rabbit red blood cell ghost method, the crude product and purified product (Substance 1).

▲ : crude extract
● : crude product
■ : purified product (Substance 1)
◉ : butylhydroxyanisole

Fig. 10 shows an oxidation curve of linoleic acid, where the procedure of autooxidation was analyzed by the rhodan iron method, using linoleic acid as a reaction substrate.

- ● - : control group
- o - : α-tocopherol group
--△-- : butylhydroxyanisole group
- ◉ - : crude extract from the sesame cell

Best Mode for Practicing the Invention

The sesame cells grown at high temperature which is used in the present invention grows rapidly at high temperature of 33 to 36 °C and contains large quantities of the antioxidative glycoside therein. Preparation of the cell containing the antioxidative glycoside through culture at such high temperature is based on the new finding as described above.

Cultivation of the sesame culture cell of the present invention which well grows at high temperature is described below.

Firstly, a germ, a root or a seed of sesame can be used. A seedling is prepared under sterile conditions and cut pieces of the germ, stalk, leaf and/or root is cultured in a solid and/or liquid medium to induce a callus cell. The obtained growing callus grows to a big callus by subculture. Then the grown callus is subjected to stationary and/or spinner culture in a solid and/or liquid medium to grow the callus cell.

As the medium, there may be used various media. As carbon sources, there may also be used polysaccharides such as oligosaccharides, starch, etc., in addition to monosaccharides such as glucose, fructose, etc., disaccharides such as maltose, sucrose, etc. As nitrogen sources, there may be used nitrate type nitrogen such as ammonium nitrate, potassium nitrate, etc., ammonia type nitrogen such as ammonium sulfate, ammonium tartarate, etc., Casamino acid, amino acid, peptone, corn steep liquor, yeast cells, yeast extract, maltose extract, etc.

In addition, there may be used vitamins such as nicotinic acid, nicotinamide, thiamine, folic acid, biotin, etc.; nucleic acid-associated substances such as inositol, adenylic acid, guanylic acid, citidylic acid, thymidylic acid, cyclic AMP, etc.; minerals such as iron, manganese, zinc, boron, iodine, potassium, cobalt, magnesium, molybdenum, phosphorus, copper, etc.

An example of basal medium is shown in Table 1.

Table 1

| | |
|---|---|
| Ammonium nitrate | 1,650 mg |
| Potassium nitrate | 1,900 |
| Calcium chloride | 440 |
| Magnesium sulfate | 370 |
| Monopotassium phosphate | 170 |
| Boric acid | 6.2 |
| Manganese sulfate | 22.3 |
| Zinc sulfate | 8.6 |
| Potassium iodide | 0.83 |
| Sodium molybdate | 0.25 |
| Cobalt chloride | 0.025 |
| Copper sulfate | 0.025 |
| Sodium ethylenediaminetetraacetate | 37.3 |
| Ferrous sulfate | 27.8 |
| Myoinositol | 100 |
| Glycine | 2 |
| Pyridoxine hydrochloride | 0.5 |
| Nicotinic acid | 0.5 |
| Thiamine hydrochloride | 0.1 |
| Sucrose | 30 g |
| Water | 1,000 ml |

pH 5.7

It is preferred to supplement auxin and cytokinin to the basal medium. As the auxin, indoleacetic acid, indolebutyric acid, naphthaleneacetic acid, 2,4-dichlorophenoxyacetic acid, etc. may be appropriately used. As the cytokinin, there may be used benzyladenine, kinetin, etc. These plant hormones or cytokinins may be used singly but the use in combination is effective. The growing callus may be cultured in a medium having the composition shown in Table 1. However, for further improving its growing nature, it is effective to supplement natural organic nutrient sources such as coconut milk, casein hydrolysate, potato extract, corn steep liquor, yeast extract, maltose extract, etc. Culture temperature may be operated between 28 and 37°C but preferably between 33 and 36°C. It is advantageous for proliferation to set a pH of the culture broth to weakly acidic (pH 5.6 to 6.0).

In order to cultivate the stably growing cells from the thus obtained sesame callus cells, a small mass of the cells are transplanted to solid plate medium solidified with gellan gum or agar and cultured for 1 to 2 weeks under growth conditions later described. In this case, it is preferred to maintain the culture temperature at 33 to 36°C.

The cell line having high growing property is selected from many cell lines of high density culture cells. A large number of small cell masses are taken out of the cell population and transplanted to fresh medium thereby to construct a larger number of the cell lines. By repeating subculture of such cell lines 5 to 10 times at high temperature of 33 to 36 °C, sesame culture cells capable of stably growing at high temperature are cultivated.

To extract the antioxidant from the growing cells obtained by cultivation, the antioxidant may be recovered by destructing the cells by biological treatment using cellulase or lysozyme, chemical treatment, mechanical treatment or ultrasonication, or combination thereof, and then extracting the antioxidant with methanol, ethanol, acetone, chloroform or other organic solvent, water, or the like, singly or as admixture of these organic solvents and water.

The activity of the antioxidant may be assayed by determining an autoxidation of linoleic acid as a reaction substrate with air according to the rhodan iron method. This is a conventional method used to evaluate peroxidation of lipid (Agricultural and Biological Chemistry, 45, 735 (1981)).

Next, collection of sesame culture cells which are cultivated according to the present invention and can grow at high temperature is described in detail, with reference to experimental steps.

1. Preparation of sesame seedling aseptically:

After thoroughly washing with water, sesame seeds are immersed in 75% ethanol aqueous solution for several seconds. The seeds are then washed with sterile water and then immersed in 0.1% benzalkonium chloride (commercially available sterilizer) for 2 to 5 minutes to sterilize microorganism adhered to the seeds. After thoroughly washing again with sterile water, the seeds are treated with 1% sodium hypochlorite (manufactured by Wako Pure Chemicals) sterilizer solution (containing 0.1% surfactant, Tween 20) for 30 minutes to completely sterilize the sesame seeds.

On the other hand, a sterilized wide-mouthed bottle with a cover (plastic-made, commercially available) is prepared. Basal medium having a composition shown in Table 1 (provided that no sucrose was added; 0.8 to 1.5% of a solidifying agent is added in the case of agar and 0.2 to 0.3% was added in the case of gellan gum) is separately sterilized in an autoclave and poured into the wide-mouthed bottle to solidify and used as solid medium for seeding. Alternatively, sterile water and sterile gauze are aseptically put in a wide-mouthed bottle, which may be used as a seeding bed. The sterilized sesame seeds are seeded on such a medium or bed for seeding by aseptic operation. When the medium or bed is kept at 28 to 30 °C in a temperature controlled room under light of a fluorescent lamp, the sterilized sesame seeds seedle without being perished. Thus, sesame seedlings of 3 to 5 cm long may be prepared in about 10 days. The seedlings are in a completely sterilized state and utilized for cultivation of growing cells.

2. Induction culture of growing cell mass derived from sesame

Media having various composition are prepared by adding naphthaleneacetic acid ($10^{-8}$ to $10^{-5}$ M) or 2,4-dichlorophenoxyacetic acid ($10^{-8}$ to $10^{-5}$ M) as auxin; benzyladenine ($10^{-6}$ to $10^{-4}$ M) or kinetin ($10^{-6}$ to $10^{-4}$ M) as cytokinin, in combination, to basal medium having the composition shown in Table 1. As a solidifying agent 0.2% of gellan gum or 0.8% of agar is added to the medium and a pH is adjusted to 5.7. Then the medium is separately charged and solidified in a Petri dish conventionally used for incubation of a microorganism. A section of the sesame seedling aseptically prepared as described above is transplanted to the medium and cultured in a temperature controlled room or box at 28 to 30 °C in the dark. Two to three weeks after the transplantation, the cells grow from the section of the sesame seedling to a mass to form callus. By subculturing the growing callus in medium having the same composition, a large callus can be cultivated.

As the basal medium used for artificial induction of callus, the medium composition (Murashige-Skoog's medium) shown in Table 1 was used. However, any medium is usable so long as it is conventionally used for plant cell culture. Basic composition of such medium is well known in the art (Manual of Plant Cell Culture, published by KODANSHA (1984)).

3. Cultivation of callus cells

The growing cell mass (callus) induced from sesame seedling is cultivated into stably growing cells in the sterile, solidified medium obtained by supplementing auxin or cytokinin such as naphthaleneacetic acid (1 to 5 x $10^{-5}$ M), benzyladenine (1 to 5 x $10^{-5}$ M), etc. to the medium having the composition shown in Table 1 and further adding thereto 0.2% of gellan gum or 0.8% of agar as a solidifying agent.

Sesame cells well grow in the dark but grow more actively in the light. It is thus observed that sesame cells well grow in the light of 3,000 to 30,000 lux, preferably 8,000 to 15,000 lux. The sesame cells may grow at temperature of 30 to 37 °C but are cultured preferably at 33 to 36 °C.

In order to cultivate cells capable of stably growing at high temperature from the sesame callus, a small mass of the cells are transplanted to solid plate medium obtained using gellan gum or agar followed by culturing for 1 to 2 weeks under the growth conditions described above.

The culture temperature in this case is set at temperature higher than that used for induction culture of callus cells. That is, the culture temperature is maintained at 33 to 36 °C, whereby the cells vigorously grown can be concentrated.

From many cell lines thus obtained, the cell line which grows vigorously is selected and many small cell masses are taken out of the cell population. The small masses are transplanted to fresh medium to prepare many cell lines. By repeating subculture of these cell lines 5 to 10 times, sesame cells which can stably grow at high temperature of 33 to 36 °C are cultivated.

4. Culture of growing cells

For cultivation of sesame cells capable of stable subculture, a medium having the composition shown in Table 1 is used but media having compositions conventionally used for culturing plant cells may also be utilized. In such a basal medium, naphthaleneacetic acid (1 to 5 x $10^{-5}$ M) and benzyladenine (1 to 5 x $10^{-5}$ M) are formulated as auxin and cytokinin, respectively. In the case of liquid culture, the medium may be used for growth culture as it is but in the case of culturing in solid medium, 0.2% of gellan gum or 0.8% of agar is added to the medium, sterilized and solidified for use. It is advantageous for the cell growth to illuminate the cells with light. Illumination may be performed with generally 3,000 to 30,000 lux, preferably 8,000 to 15,000 lux. The cells grow at temperature of 28 to 37 °C, preferably 33 to 36 °C. For liquid culture, shake culture method or agitation and aeration culture method which is used for culturing ordinary microorganisms is adopted, but is preferably operated under milder conditions than in culturing microorganism. An amount of oxygen required is extremely smaller than that required for culturing microorganisms. It is thus preferred for cell growth to stir to such a degree that the cells do not deposit at the bottom of culture broth, while aerating slightly.

For more effective growth culture, it is preferred to maintain a pH of the culture broth at 5.6 to 5.8.

In ordinary growth culture, incubation is completed in 1 to 2 weeks. It is thus possible to recover the cells from the culture broth in conventional manner such as centrifugation, etc. In the case of solid culture, the proliferated cell mass may be readily recovered.

Thus, sesame cells capable of producing in industrial scale are cultivated and proliferated in large quantities.

Fig. 1 shows relationship between growth of the thus obtained sesame culture cells and culture temperature. The growth at 35 to 36 °C is about twice or more than that at temperature of 25 to 28 °C which is generally used for culturing plant cells. The growth rate is also fast. Therefore, the sesame culture cells can be effectively used in culturing plant cells industrially.

5. Extract of antioxidant and its assay

The growing callus cell obtained as described above is broken into fine pieces with a blender, etc. and then macerated together with quartz sand. The macerated cells are extracted with solvent such as methanol, etc., dehydrated with anhydrous sodium sulfate and evaporated to dryness at 30 to 35 °C. The residue is again dissolved in methanol to give a fraction containing the antioxidant.

Next, a method for assaying the antioxidative activity is described. The method comprises using linoleic acid as a reaction substrate and utilizing the rhodan iron method often used to determine the degree of oxidation of oils and fats. That is, the method comprises oxidizing divalent iron ions to trivalent iron ions with peroxides formed by autoxidation of oils and fats; reacting the trivalent iron ions with ammonium thiocyanate to form red rhodan iron; and measuring absorbance of the rhodan iron, whereby an amount of the peroxides of oils and fats is determined (Agricultural and Biological Chemistry, 45, 735 (1981)).

Furthermore, a so called red cell ghost method utilizing peroxidation of rabbit red cell membrane lipid was also used as a method for assay, which is closer to the *in vivo* system, than in the rhodan iron method.

From rabbit red blood cells, their membrane alone is prepared and called erythrocyte membrane.

After t-butylhydroxy peroxide is added to the erythrocyte membrane ghost as an initiator of oxidation to accelerate peroxidation of phospholipid in the erythrocyte membrane, the produced malonedialdehyde (strongly carcinogenic substance) or its analog is reacted with thiobarbituric acid to form a color compound

and its absorbance is measured (Biochemistry, 78, 6858 (1981)).

In any of the methods, a degree of the color formed with thiobarbituric acid changes in association with the degree of antioxidative activity in the sample added to the reaction system. Thus, by contrasting with the control group containing no antioxidant, the antioxidative activity in the sample can be determined.

6. Extract, isolation and structural analysis of antioxidative glycoside

The cells grown by liquid culture may be readily recovered by gravity fractionation or centrifugation. Ethyl alcohol is added to the thus obtained cells in a final concentration of 80%. The antioxidative glycoside is extracted from the mixture with a mechanically stirring homogenizer. After the extract is recovered by centrifugation, the remained cells are again extracted with 80% ethyl alcohol. The resulting extract is concentrated under reduced pressure and the concentrate is evaporated to dryness to give the syrup-like, yellowish brown crude extract of antioxidant.

The crude extract is subjected to adsorption chromatography using, e.g., Amberlite XAD-II to adsorb the antioxidant onto the resin, which is then eluted with a solvent mixture of water and methanol to give the fraction having antioxidative activity. The fraction eluted with 40% methanol aqueous solution is collected and concentrated under reduced pressure to give representative components of the antioxidant contained in the sesame culture cells.

By isolation and assay of the components by means of high performance liquid chromatography conventionally used in the field of natural chemistry, the degree of purification of the desired components is determined. High performance liquid chromatogram of the fraction eluted from the alcohol extract of sesame culture cells with 40% methanol aqueous solution by adsorption chromatography is shown in Fig. 2.

Among many peaks, Peak 1 (peak showing an elution time of 6 minutes) is further purified as one of the peaks having antioxidative activity. In this purification, high performance liquid chromatography conventionally used to purify natural materials is repeatedly used to purify the desired products in sequence. Purification degree of the substance having Peak 1, which was purified by high performance liquid chromatography, is shown in Fig. 3. This antioxidant is named Substance 1.

Instrumental analysis on Substance 1 isolated as a single compound was performed with apparatuses ordinarily used to determine its chemical structure.

Firstly, UV absorption spectrum of Substance 1 is shown in Fig. 4. Analysis chart of Substance 1 by high speed electron bombardment mass spectrum (FAB-MS) is shown in Fig. 5. Based thereon, the molecular ion peak ($M^+$ + 1) is 625 and the molecular weight of Substance 1 is 624. Further by nuclear magnetic resonance (NMR), the presence of olefin and aromatic ring and the position of substituents are determined (Fig. 6).

On the other hand, Substance 1 is hydrolyzed with hydrochloric acid to prepare methyl ester of aglicon molecule to confirm its structural analysis. From the analysis by nuclear magnetic resonance and electron impact mass spectrum (EI-MS) and UV absorption curve, etc., caffeic acid methyl ester having a molecular weight of 194 was identified so that aglicon of Substance 1 was attributed to caffeic acid.

The color forming reaction of sugar indicates that Substance 1 has a glycoside bound to sugar molecule. In order to determine its structure, the methylated compound was prepared using diazomethane and its structure was analyzed by protonic nuclear magnetic resonance and carbon 13 nuclear magnetic resonance. Their spectra are shown in Figs. 7 and 8, respectively.

Using such methods for the structural analysis of natural matters, the structure of Substance 1 was determined to be:

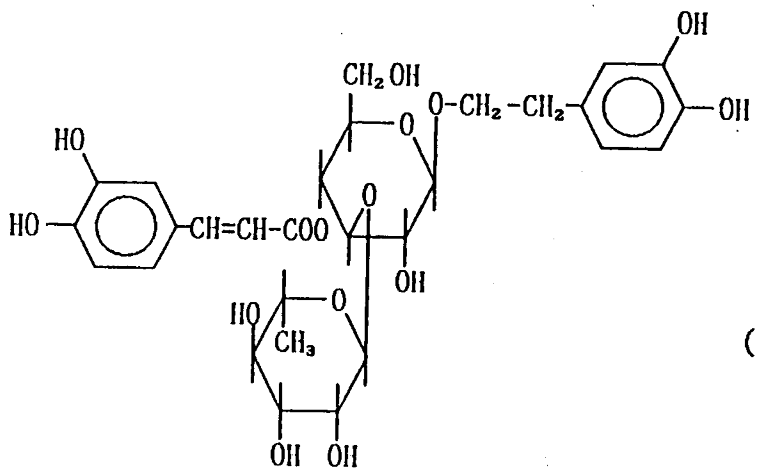

(I)

7. Antioxidative activity of antioxidative glycoside

With respect to the crude extract obtained by extracting from the sesame culture cells with 80% ethanol, a crude product obtained by eluting the crude extract with 40% methanol aqueous solution by adsorption chromatography using Amberlite XAD-II and further Substance 1 obtained by purifying the crude product by high performance liquid chromatography to the degree until the single peak is obtained, the antioxidative activity was determined by the rabbit red cell ghost method described above. The results are shown in Fig. 9. The abscissa represents a concentration of the sample added to the reaction system in the analysis and the coordinate represents a degree of the color formation with thiobarbituric acid in terms of a relative ratio when the color formation in the control group without antioxidant was 100%.
Comparison was made with the control group using commercially available synthetic antioxidant, butylhydroxyanisole (BHA).

As described above, it is revealed that the antioxidative glycoside obtained by extracting from the sesame culture cells had antioxidative activity almost equivalent to that of BHA.

Where Substance 1 is industrially used as the antioxidant, it is advantageous to use the purified antioxidant or the crude product depending upon purpose. As shown in Fig. 9, there is no substantial difference between the purified compound and the crude product obtained by adsorption chromatography. Strictly speaking, it is considered that the antioxidative activity would be appreciated also in the components other than Substance 1 which are contained in the crude product, or the co-present substances would have an accelerating action (synergistic action) of the antioxidative activity. This sufficiently suggests that the crude products would be usable.

Furthermore, the fact that the substance has glycoside structure indicates that it has characteristic property of between water solubility and fat solubility. Use range of the antioxidant is broadened, as compared to water-soluble vitamin C, fat-soluble vitamin E, butylhydroxyanisole (BHA), butylhydroxytoluene (BHT), etc. Also in vivo activity, its advantage is greatly expected.

Hereinafter the present invention is illustratively described with reference to the examples and test examples, but is not deemed to be limited thereto.

Each cell in accordance with the present invention can be readily obtained by using ordinary sesame plant and treating the sesame plant body according to the procedures described above and the procedures of the examples later described. It was confirmed that they are sufficiently reproductive. The starting material is readily accessible without any difficulty and its treatment involves no particular difficulty. Therefore, anyone can make easy access to the cells in accordance with the present invention.

Example 1

(1) Seeds of sesame (Sesamum indicum L.) prepared were immersed in 75% ethanol for several seconds followed by washing twice with sterile distilled water. The seeds were then immersed in 0.1% benzalkonium chloride solution (manufactured by Amakasu Chemical Industry Co., Ltd.) for 2 minutes. After thoroughly wahsing 3 times with sterile distilled water, the seeds were immersed in a sterilizer solution containing 1% sodium hypochlorite (Wako Pure Chemicals) and 0.1% Tween 20 (Wako Pure Chemicals) for 30 minutes. After washing with sterile water, sterilized sesame seeds were prepared.

Sterile water and sterile gauze were put in a plastic container (manufactured by Flow Laboratories Co., Ltd.) for plant culture and the previously sterilized sesame seeds were seeded thereon. The seeds were allowed to stand for 2 weeks under a fluorescent light of 20 W in a temperature controlled room at 30 ° C. Seedlings of 5 to 7 cm long were thus obtained.

(2) Two liters of medium having the composition shown in Table 1 were prepared and 100 ml of each aliquot was supplemented with 0.2% of gellan gum (manufactured by San-Ei Chemical Industry) and cytokinin and auxin under the experimental conditions as shown in Table 2, which were made induction media of growing cell mass (callus). These media were sterilized in an autoclave at 120 ° C for 10 minutes.

These media were separately charged in 3 each of Petri dish having a diameter of 10 cm by 30 ml each and solidified at room temperature.

The sesame seedlings prepared in (1) were cut at the stalk and leaves into sections of 5 to 7 mm by aseptic operation and transplanted to the solid media in Petri dishes. In order to prevent moisture from being evaporated, sealing was made with Parafilm (manufactured by American Can Co., Ltd.). By allowing to stand in a temperature controlled room of 28 to 30 ° C in the dark for 3 weeks, callus was induced from the sections of sesame seedlings. The results are shown in Table 2.

Table 2

| Cytokinin | Auxin | State of Callus Induction |
|---|---|---|
| Benzyladenine | Naphthaleneacetic acid | |
| $1 \times 10^{-5}$ M | $5 \times 10^{-5}$ M | ++++ |
| | 1 | +++ |
| | 0.1 | ++ |
| | 0.01 | - |
| | 2,4-Dichlorophenoxy-acetic acid | |
| | $5 \times 10^{-5}$ M | - |
| | 1 | + |
| | 0.1 | ++ |
| | 0.01 | ++++ |
| Kinetin | Naphthaleneacetic acid | |
| $1 \times 10^{-5}$ M | $5 \times 10^{-5}$ M | ++++ |
| | 1 | ++++ |
| | 0.1 | +++ |
| | 0.01 | - |
| | 2,4-Dichlorophenoxy-acetic acid | |
| | $5 \times 10^{-5}$ M | + |
| | 1 | + |
| | 0.1 | +++ |
| | 0.01 | ++++ |

+: amount of callus induced; the larger the number of symbol +, the better the results

-: no callus induction

(3) After adding 0.2% gellan gum, $5 \times 10^{-5}$ M of naphthaleneacetic acid and $1 \times 10^{-5}$ M of benzyladenine to basal medium having the composition of Table 1, 600 ml of medium was sterilized and prepared in a manner similar to (1). The medium was separately charged in 20 plastic Petri dishes by 30 ml each and solidified. The growing callus obtained by induction culture in (1) was transplanted to Petri dish by 4 each per dish. Culture was performed at 33 to 36 ° C for 2 weeks in a plant cell culture device

under light of 12,000 lux. The cell mass showing a good growth property was selected and made seed cells. Subculture of the seed cells was repeated 4 times at 33 to 36 °C. Thus, the culture cell capable of stably growing at high temperature was cultivated. The cell was named $N_5B_5$S-HT (sesame culture cell subcultured with 5 x $10^{-5}$ M of naphthaleneacetic acid and 1 x $10^{-5}$ M of benzyladenine).

(4) After adding 0.2% gellan gum, 5 x $10^{-5}$ M of naphthaleneacetic acid and 1 x $10^{-5}$ M of benzyladenine to basal medium having the composition of Table 1, 1 liter of the medium was sterilized and prepared in a manner similar to (1). The medium was separately charged in a glass container for plant cell culture having a diameter of 4 cm and depth of 13 cm by 40 ml each and then solidified. A 5-7 mm section of sesame growing cell $N_5B_5$S-HT cultivated in (3) by subculture was transplanted and cultured at 35 to 36 °C for 10 days in a plant cell culture device under light of 12,000 lux. As the result, the cells grown in the culture container weight 14.5 g in average.

After 60 g of the cells was taken in a mortar, 6 g of quartz sand was added thereto. After maceration for 5 minutes, 200 ml of 80% ethanol aqueous solution was added to the mixture and well stirred. The antioxidant was then extracted. The extract was centrifuged (2,500 rpm, 10 minutes) and the supernatant was collected. After 200 ml of 80% ethanol aqueous solution was added to the cell debris, the mixture was extracted. The extracts obtained by extraction with ethanol aqueous solution 3 times were collected and evaporated at 40 °C to dryness with a rotary evaporator to give 4.8 g of the yellowish brown extract.

Example 2

After 3 liters of medium having the composition shown in Table 1 was charged in a plant cell culture tank of 5 liter volume equipped with aeration and agitation devices, the medium was sterilized at 120 °C for 20 minutes in an autoclave under pressure. Separately, 60 ml of medium having the composition shown in Table 1 was charged in an Erlenmeyer's flask of 300 ml and sterilized in a similar manner. Seeds of sesame culture cells were added to the medium and shake-cultured at 35 °C under a fluorescent light under the shake number of 60 rpm. The culture cells corresponding to 5 flasks cultured for 7 days were recovered by aseptic operation and inoculated in the plant cell culture tank. Culture conditions of the plant cell culture tank were: agitation speed of 30 rpm, pH of 5.7 ± 0.1, aeration rate of 1.5 liter/min, light exposure of 8,000 lux, and temperature of 35 °C for 10 days. After completion of the culture, the culture broth was centrifuged to recover the cells. On a dry weight basis, 43 g of the cells were obtained.

Using 500 g of the sesame cells obtained by culture, the antioxidant was extracted and purified. That is, 1.9 liter of ethanol was added to the sesame cells. While stirring with a homogenizer, extraction was performed for 20 minutes. Then, the cells and the extract were separated from each other through a filter. After 2 liters of 80% ethanol water was added to the cell debris, extraction was performed for 20 minutes in a similar manner. The cell debris was then isolated through a filter and further extracted with 2 liters of 80% ethanol.

The extracts were combined and concentrated at 40 °C under reduced pressure to give 11.4 g of brown crude extract.

Amberlite XAD-II used for adsorption chromatography was packed in a glass-made column having a diameter of 5 cm and a length of 30 cm. Water was passed through the column to equilibrate. To the column was overlaid 5 g of the extract in such a state that the resin was adsorbed on the upper part of the column. By stepwise elution with water and with methanol while sequentially increasing its concentration, the desired substance was eluted. The fraction eluted with 40% methanol was collected and concentrated at 40 °C under reduced pressure to give 210 mg of the yellowish brown crude product. High performance liquid chromatography of the crude product was repeated until the single peak was obtained. As the result, 21 mg of the purified substance was obtained.

Among these crude components, the antioxidative activity of the crude product and the purified compound was determined by the rabbit red cell ghost method. As shown in Fig. 9, the antioxidative activity as potent as butylhydroxyanisole (BHA) was noted.

Test Example 1

Excellent antioxidative activity of the antioxidant prepared by the present invention was confirmed as follows.

The extract, 100 mg, obtained in the examples was dissolved in 100 ml of 80% ethanol aqueous solution (in a concentration of 1 mg/ml) to make an analytical sample of the antioxidative activity. Using 1 ml of the above solution as a sample, the degree of prevention of linoleic acid from autoxidation was determined by the rhodan iron method. As the result, it was confirmed that the antioxidant having a high

activity equivalent to that of α-tocopherol (0.2 mg) or butylhydoxyanisole (BHA, 0.2 mg) was contained in the fraction (1 mg) extracted from the sesame culture cells, as is also clear from Fig. 10.

INDUSTRIAL APPLICABILITY

The glycoside of the present invention having the antioxidative activity exhibits an excellent antioxidative property and is extracted from the cells obtained by culturing sesame cell grown at high temperature. Therefore, according to the present invention, antioxidative glycosides derived from natural matters can be supplied systematically in large quantities. Thus, the present invention may be utilized as foods, drugs and cosmetics.

**Claims**

1. A process for preparing glycoside shown by the following structural formula (I) which comprises culturing growing cells derived from the plant body of sesame (Sesamum indicum L.) in a medium and preparing said glycoside from the cultured matters.

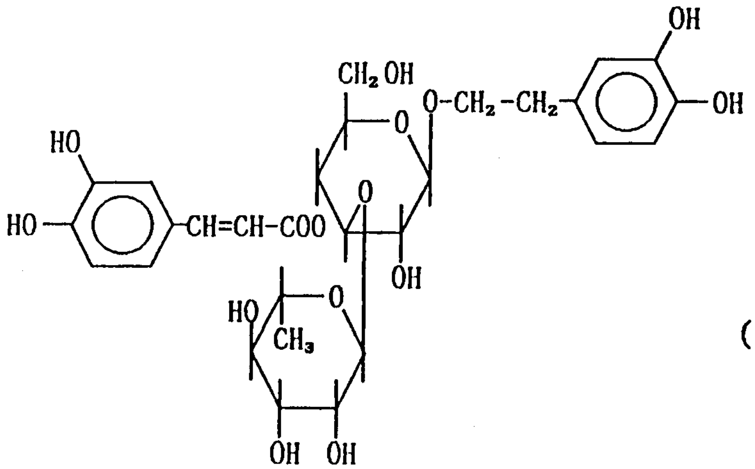

(I)

2. A process for preparing antioxidative glycoside shown by structural formula (I) which comprises culturing in a medium cells grown at high temperature derived from the plant body of sesame (Sesamum indicum L.) and preparing said glycoside from the medium.

3. An antioxidant comprising glycoside shown by structural formula (I) as an effective ingredient.

FIG. 1

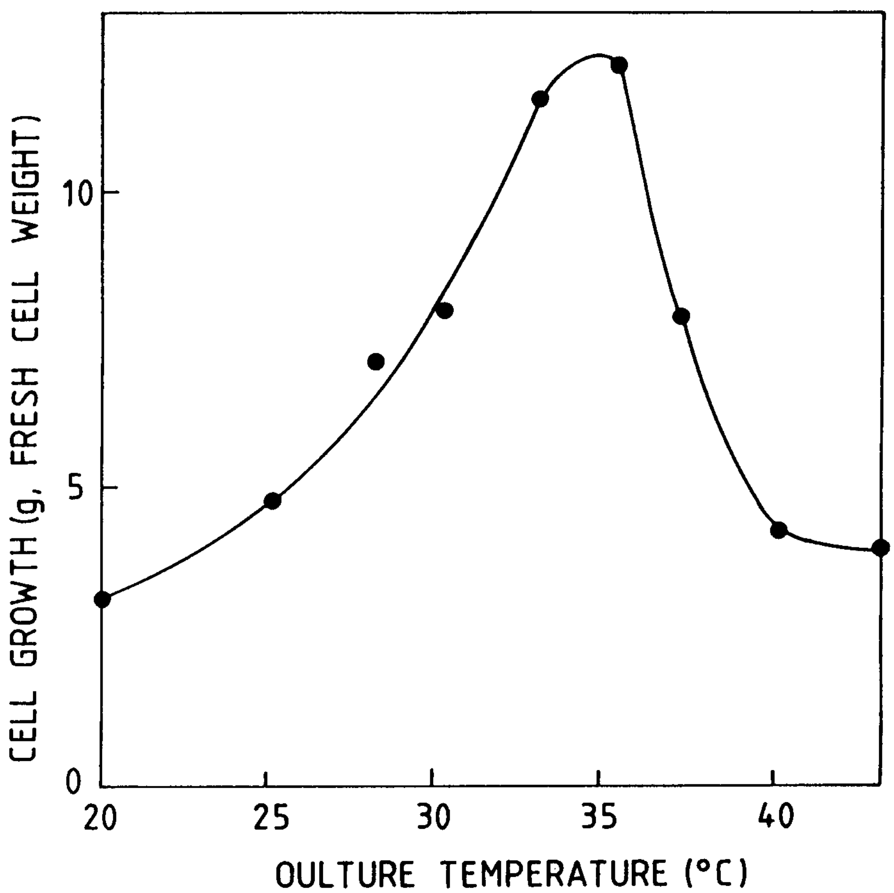
CELL GROWTH (g, FRESH CELL WEIGHT)
10
5
0
20
25
30
35
40
OULTURE TEMPERATURE (°C)

FIG. 2

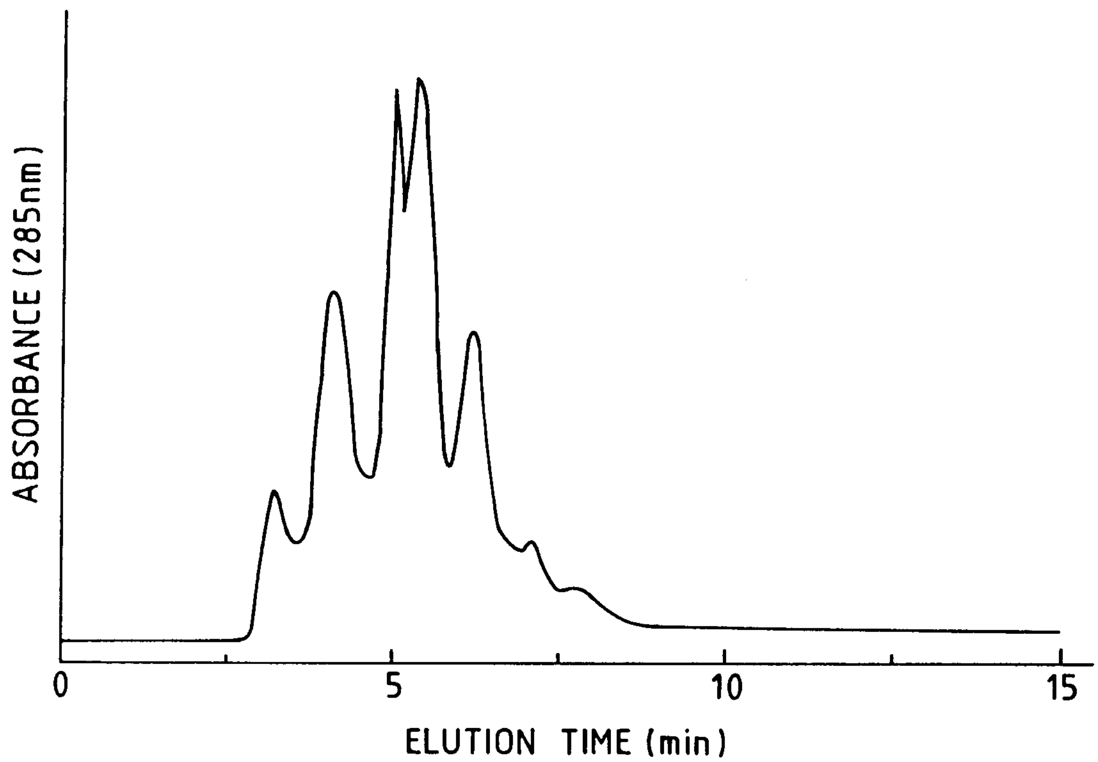

FIG. 3

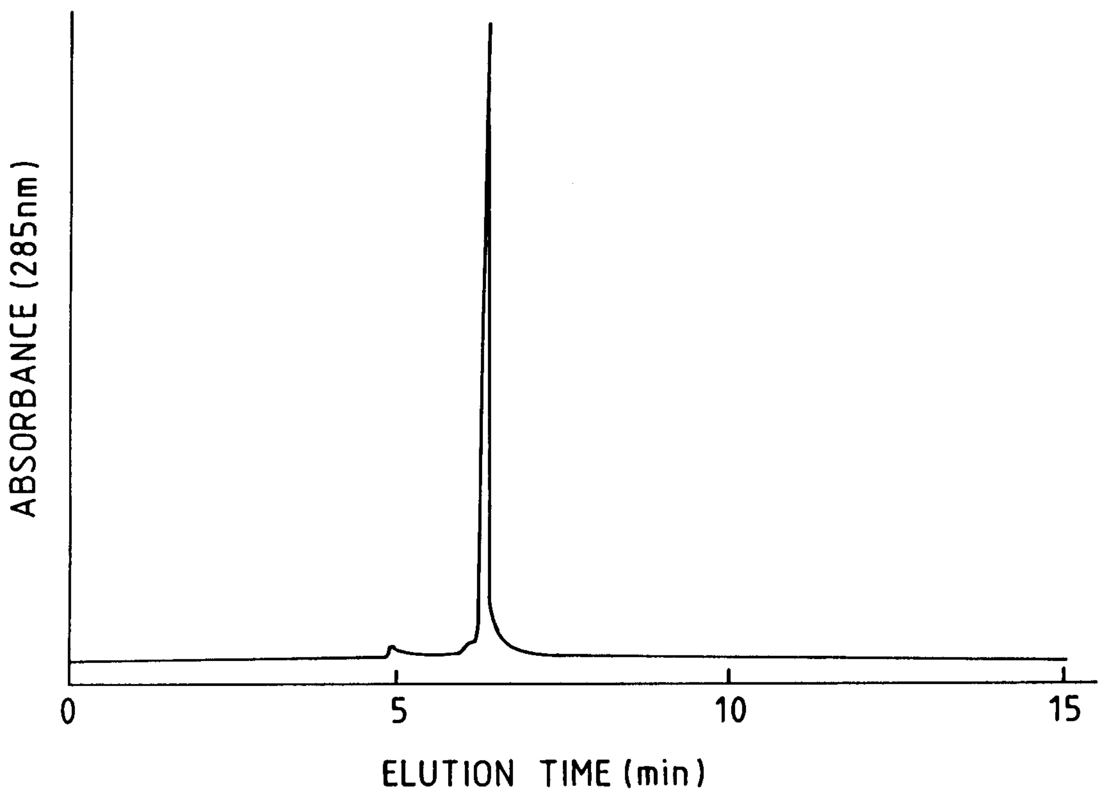

FIG. 4

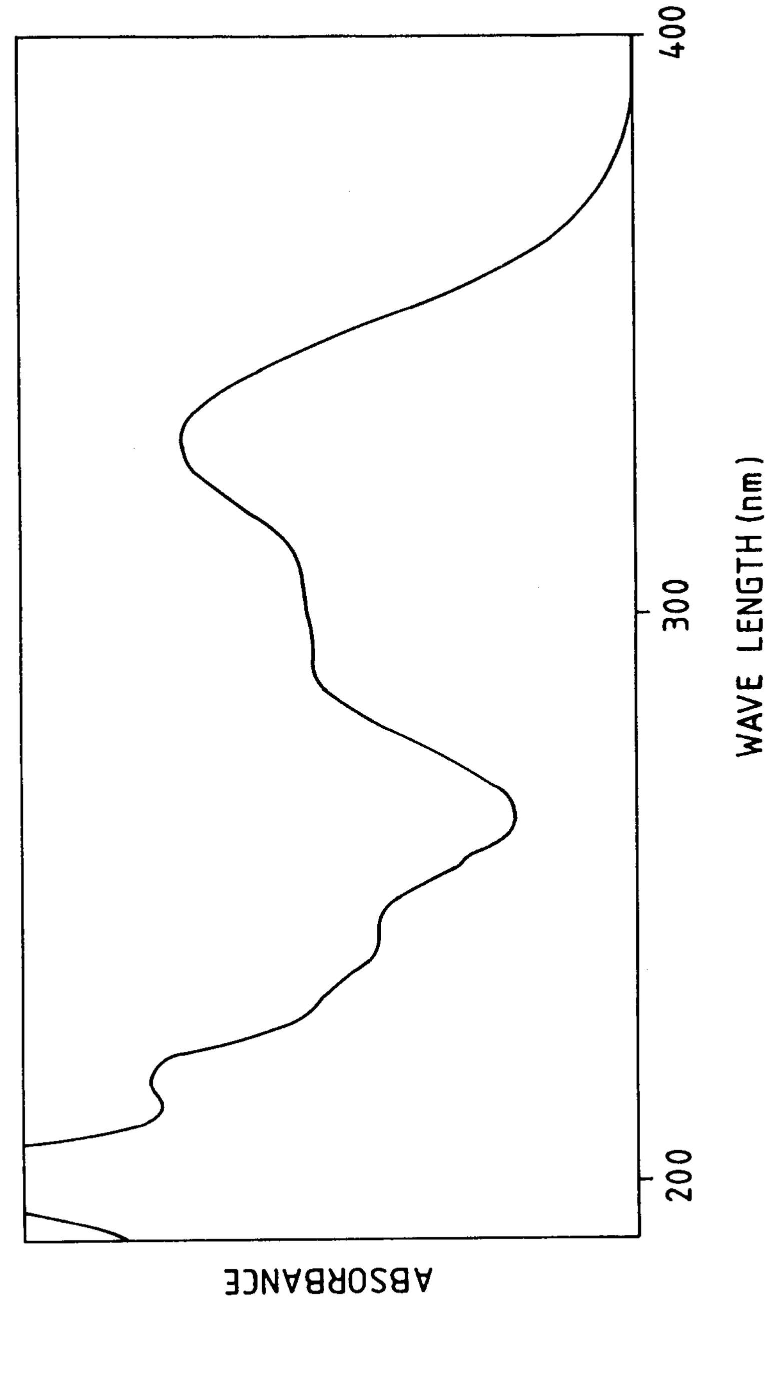

FIG. 5

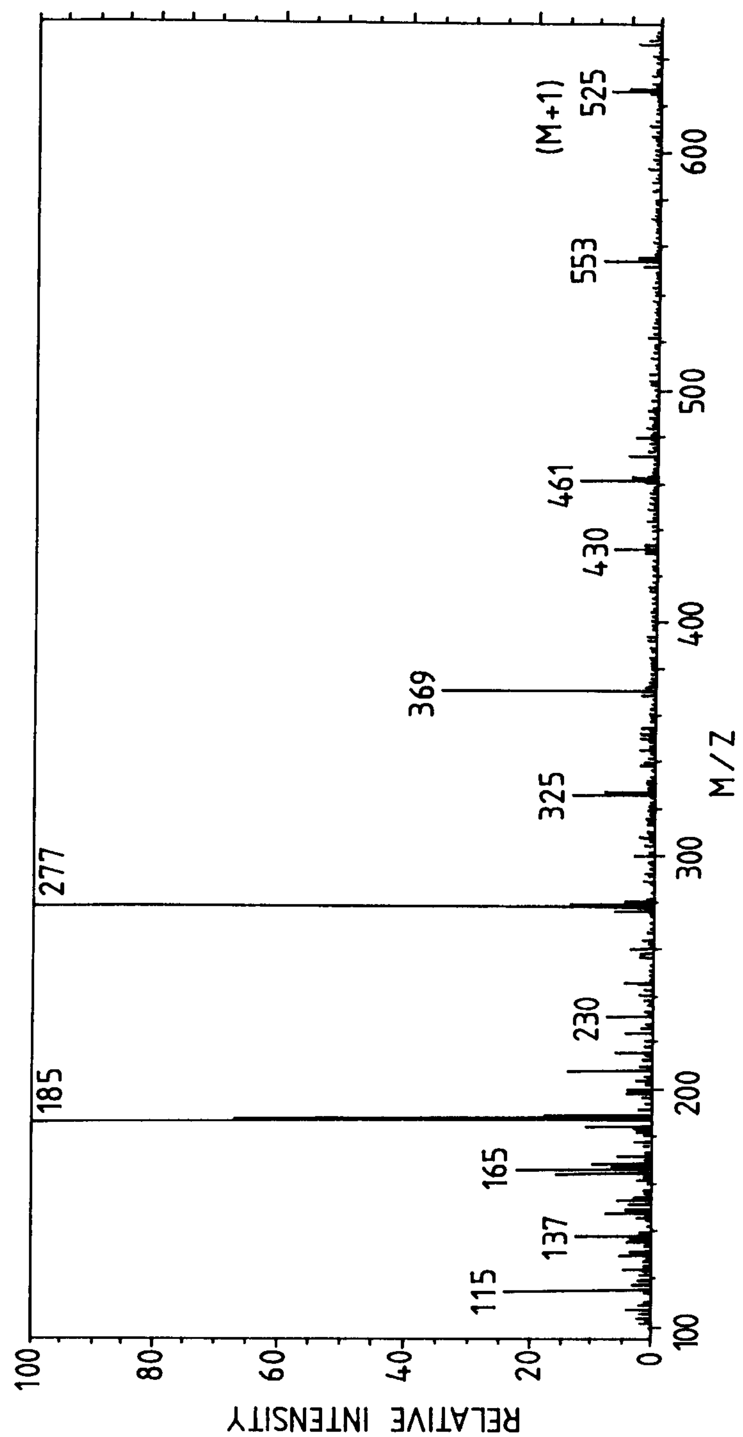
RELATIVE INTENSITY
100
80
60
40
20
0
100
200
300
400
500
600
M/Z
115
137
165
185
230
277
325
369
430
461
553
(M+1)
525

FIG. 6

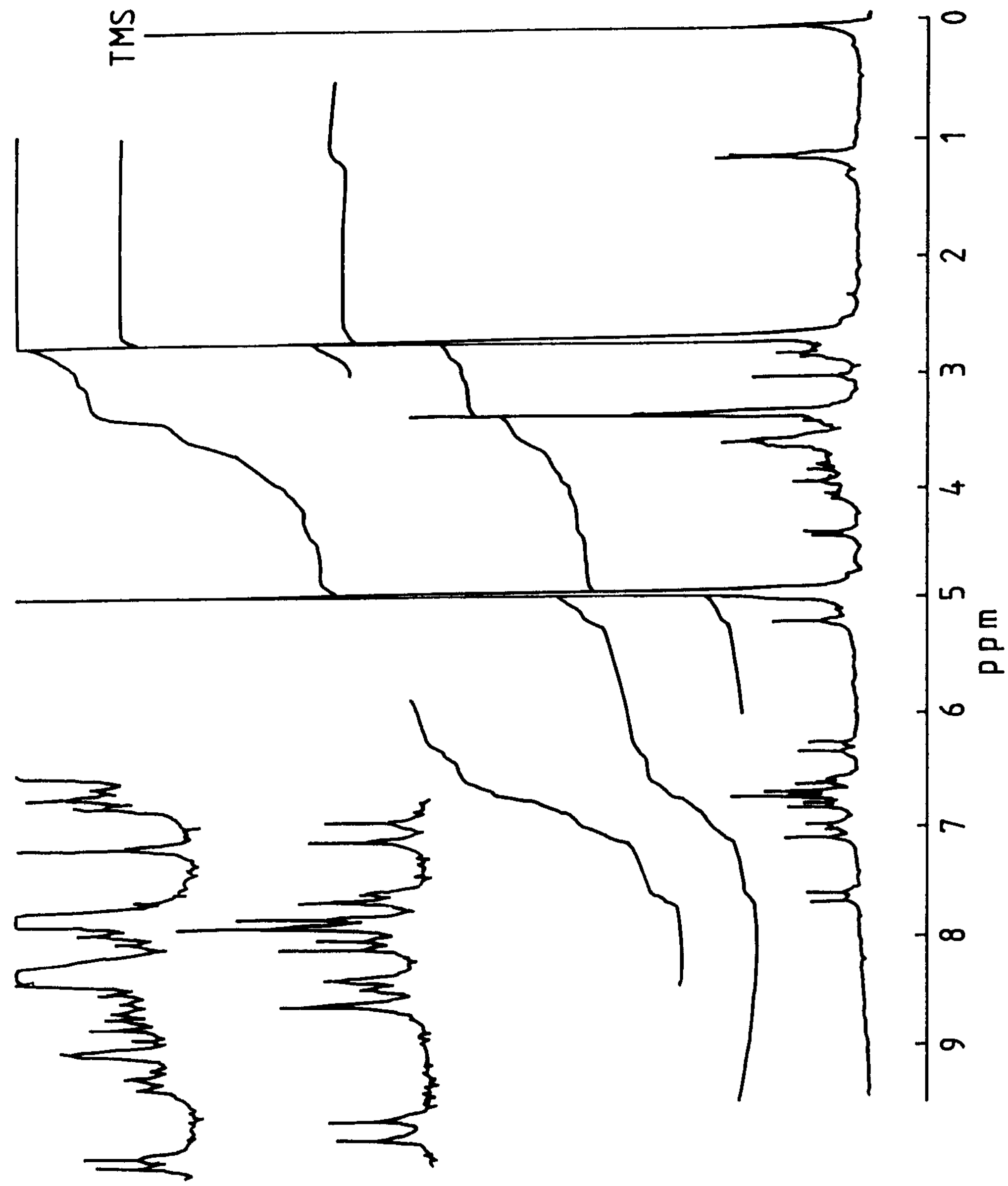

FIG. 7

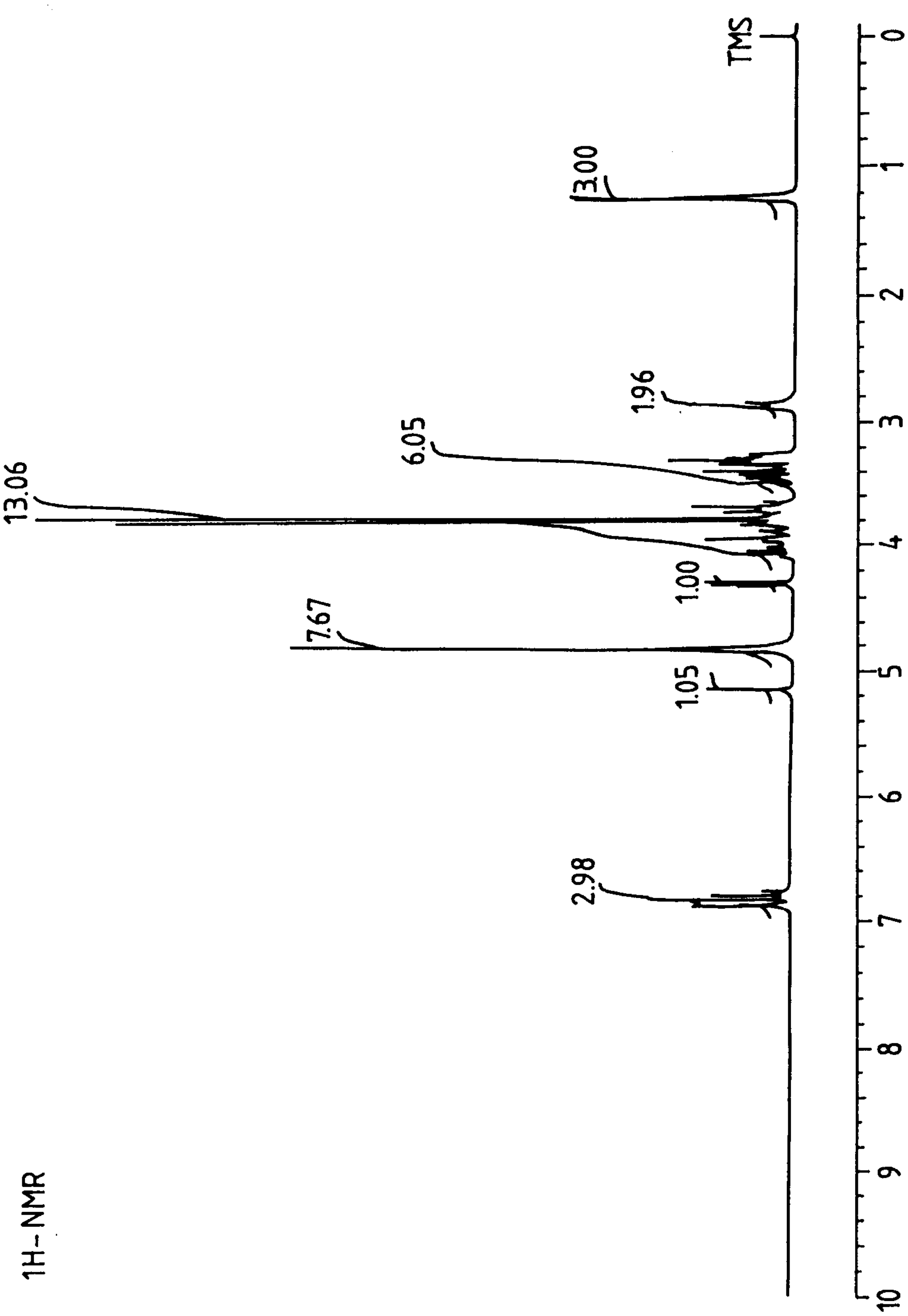

FIG. 8

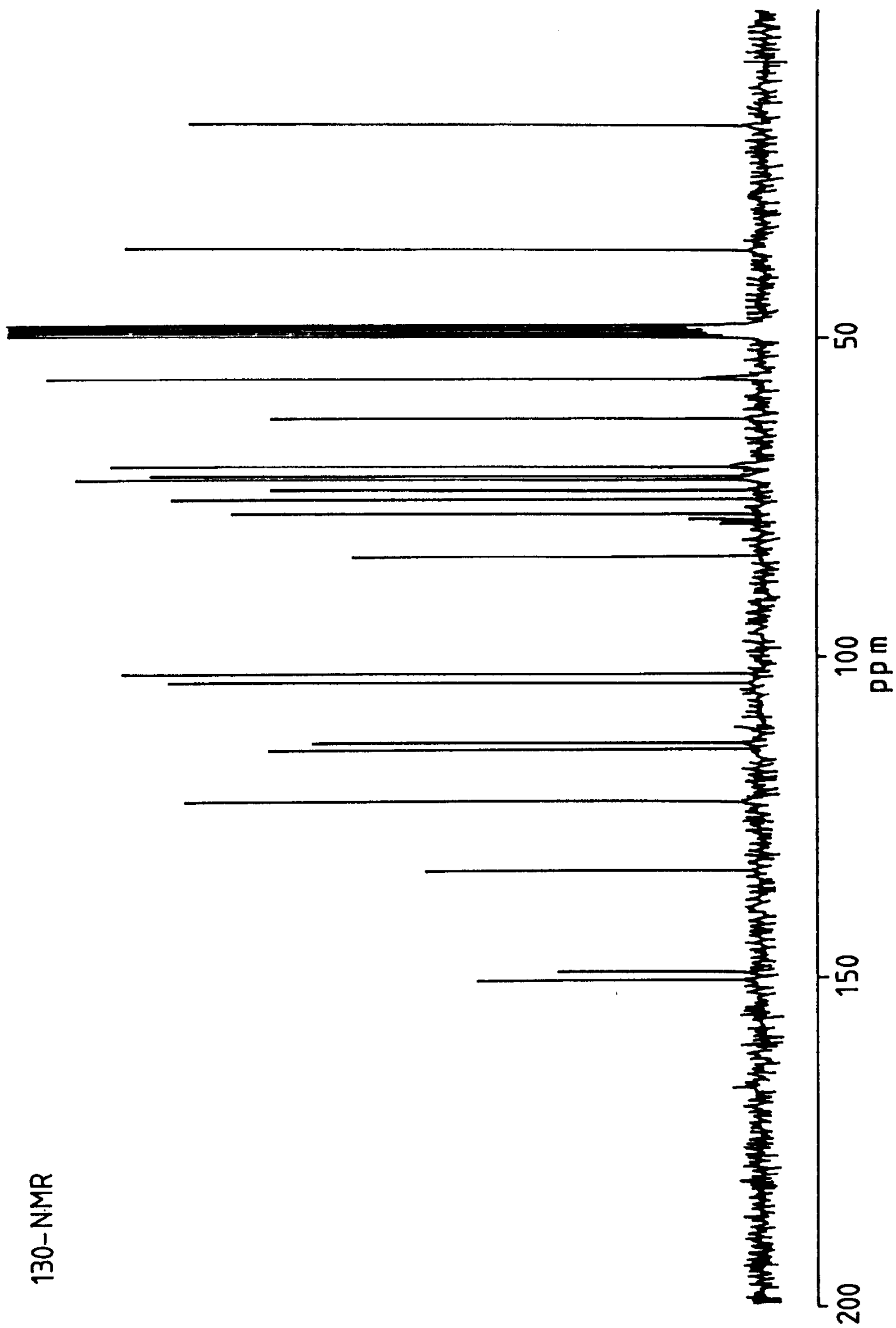

FIG. 9

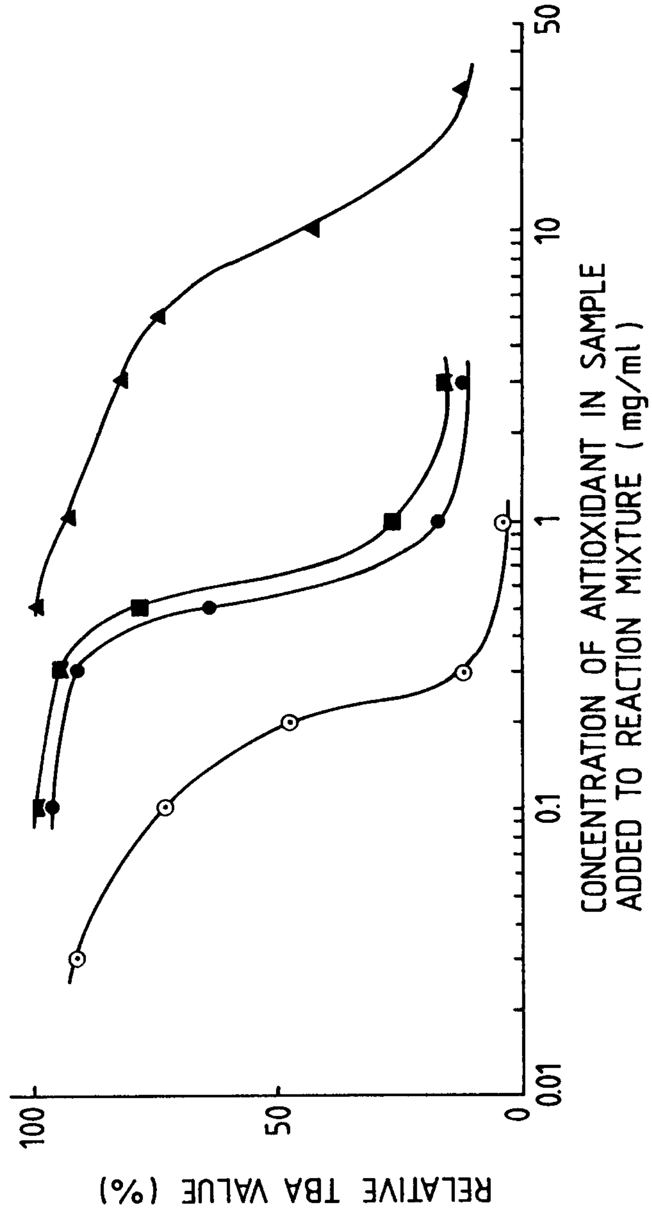
RELATIVE TBA VALUE (%)
100
50
0
0.01
0.1
1
10
50
CONCENTRATION OF ANTIOXIDANT IN SAMPLE
ADDED TO REACTION MIXTURE ( mg/ml )

FIG. 10

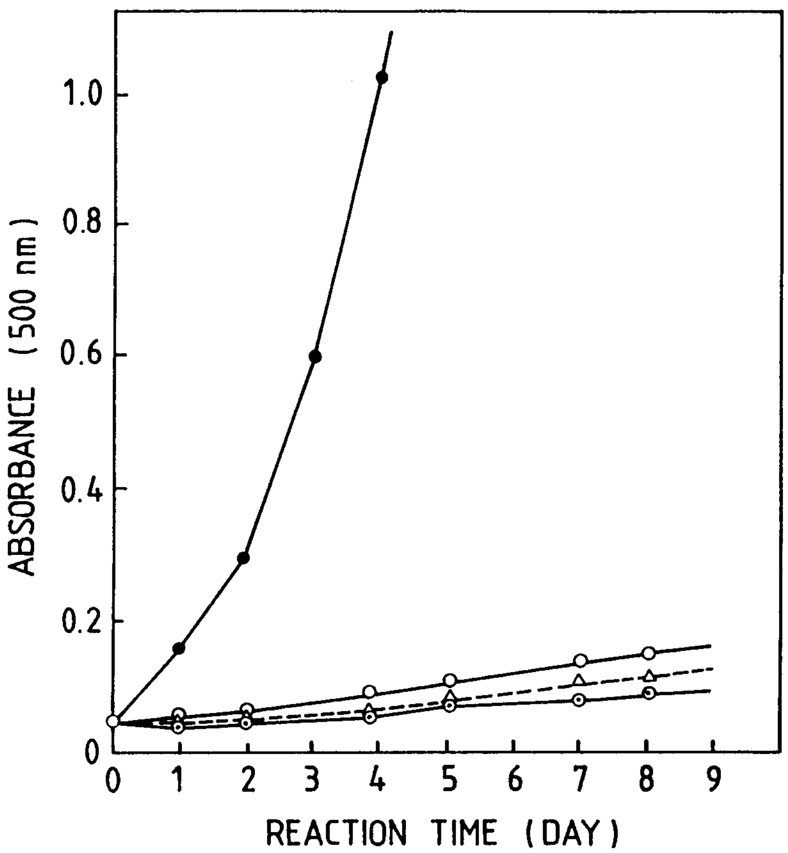

1.0
0.8
0.6
0.4
0.2
0
ABSORBANCE (500 nm)
0
1
2
3
4
5
6
7
8
9
REACTION TIME (DAY)

# INTERNATIONAL SEARCH REPORT

International Application No PCT/JP91/00199

**I. CLASSIFICATION OF SUBJECT MATTER** (if several classification symbols apply, indicate all) [6]

According to International Patent Classification (IPC) or to both National Classification and IPC

Int. Cl[5] C12P19/44, C09K15/08//(C12P19/44, C12R1:91)

**II. FIELDS SEARCHED**

Minimum Documentation Searched [7]

| Classification System | Classification Symbols |
|---|---|
| IPC | C12P19/44, C12N5/04, C09K15/08 |

Documentation Searched other than Minimum Documentation to the Extent that such Documents are Included in the Fields Searched [8]

Chemical Abstracts Data Base (CA)
Biological Abstracts Data Base (BIOSIS)

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | Phytochemistry, Vol.25, No.7, (1986), Yukihiro Shoyama et al. "Four caffeoyl glycosides from callus tissue of Rehmannia glutinosa" P. 1633-1636 | 1-3 |
| A | JP, A, 63-207380 (Kobe Steel, Ltd.), August 26, 1988 (26. 08. 88), (Family: none) | 1-3 |
| A | JP, A, 1-215282 (Kobe Steel, Ltd.), August 29, 1989 (29. 08. 89), (Family: none) | 1-3 |

* Special categories of cited documents: [10]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search | Date of Mailing of this International Search Report |
|---|---|
| May 7, 1991 (07. 05. 91) | May 20, 1991 (20. 05. 91) |
| **International Searching Authority** | **Signature of Authorized Officer** |
| Japanese Patent Office | |